# EUROPEAN PATENT APPLICATION

(11) **EP 2 754 445 A2**
(43) Date of publication of application: **16.07.2014**
(21) Application number: 14163272.9
(22) Date of filing: 17.09.2008
(51) Int. Cl.: A61K 33/30, A61K 33/34, A61K 33/38, A61K 45/06, A61P 43/00, A61N 1/20, H01M 6/26, A61N 1/30

(54) **Electricity-generating particulates for use in excessive sweating**

(30) Priority: 28.09.2007 US 975927 P
(62) Divisional of application: 08835165.5
(71) Applicant: Johnson & Johnson Consumer Companies Inc., Skillman, NJ 08558 (US)
(72) Inventor: Chantalat, Jeannette, Pennington, NJ 08534 (US); Liu, Jue-Chen, Belle Mead, NJ 08502 (US); Hauschild, James E., Cranbury, NJ 08512 (US); Ming, Xintian, Bridgewater, NJ 08807 (US); Southall, Michael, Lawrenceville, NJ 08648 (US); Sun, Ying, Belle Mead, NJ 08502 (US)
(74) Representative: Carpmaels & Ransford LLP

(57) **Abstract**

The invention features a galvanic particulate, for use in a method of excessive sweating, including a first conductive material and a second conductive material, wherein both the first conductive material and the second conductive material are exposed on the surface of the particulate, wherein the particle size of the particulate is from about 10 nanometers to about 100 micrometers, wherein the second conductive material comprises from about 0.01 percent to about 10 percent, by weight, of the total weight of the particulate, and wherein the difference of the standard potentials of the first conductive material and the second conductive material is at least about 0.2 V.

## Description

### BACKGROUND OF THE INVENTION

Using a galvanic couple as the power source in iontophoresis patch devices is well known in the art. See e.g., U.S. Patent Nos. 5,147,297, 5,162,043, 5,298,017, 5,326,341, 5,405,317, 5,685,837, 6,584,349, 6,421, 561, and 6,653,014 and U.S. Patent Applications 2004/0267237 and 2004/0138712. The galvanic couple is made from dissimilar metals, such as a zinc donor electrode and a silver chloride counter electrode. Some of these galvanic couple powered iontophoresis patch devices activate automatically when body tissue and/or fluids form a complete circuit with the galvanic system to generate the electricity. These devices are often applied to the human body in order to provide an intended benefit, such as electric stimulation, enhanced healing, or antimicrobial treatment.

Although aforementioned galvanic patches as drug delivery devices are useful therapeutic products, they can be cumbersome to use and expensive to manufacture. It is the intent of the present invention of the present invention to overcome these shortcomings by providing galvanic particulates.

### SUMMARY OF THE INVENTION

In one aspect, the invention features a galvanic particulate including a first conductive material and a second conductive material, wherein both the first conductive material and the second conductive material are exposed on the surface of the particulate, wherein the particle size of the particulate is from about 10 nanometers to about 100 micrometers, wherein the second conductive material comprises from about 0.01 percent to about 10 percent, by weight, of the total weight of the particulate, and wherein the difference of the standard potentials of the first conductive material and the second conductive material is at least about 0.2 V.

In another aspect, the invention features a method of manufacturing the particulate of the invention by contacting a particulate of the first conductive material with a solution comprising a salt of the the second conductive material.

In another aspect, the invention features an ingestible composition containing a particulate of the invention and a bio-absorbable polymer.

In another aspect, the invention features an oral dosage form comprising a particulate of the invention and a pharmaceutically acceptable carrier.

In another aspect, the invention features a method of treating a gastrointestinal disorder by orally administering a particulate of the present invention.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, a percentage refers to a percentage by weight (i.e., %(W/W)).

### Definitions

What is meant by a "product" is a product containing the galvanic particulates (or a composition containing the galvanic particulates) in finished packaged form. In one embodiment, the product contains instructions directing the user ingest, topically apply, or otherwise administer the galvanic particulates or composition (e.g., to treat a skin condition). Such instructions may be printed on the outside of the product, a label insert, or on any additional packaging.

In one aspect, the present invention features promoting the galvanic particulates or composition containing the galvanic particulates of the present invention for an intended use. What is meant by "promoting" is promoting, advertising, or marketing. Examples of promoting include, but are not limited to, written, visual, or verbal statements made on the product or in stores, magazines, newspaper, radio, television, internet, and the like.

As used herein, "pharmaceutically-acceptable" means that the ingredients which the term describes are suitable for its intended use (e.g., suitable of ingestion or contact with the skin or mucosa) without undue toxicity, incompatibility, instability, irritation, allergic response, and the like.

As used herein, "safe and effective amount" means an amount of the ingredient or the composition sufficient to provide the desired benefit at a desired level, but low enough to avoid serious side effects. The safe and effective amount of the ingredient or composition will vary with the area being treated, the age of the end user, the duration and nature of the treatment, the specific ingredient or composition employed, the particular pharmaceutically-acceptable carrier utilized, and like factors.

As used herein, the term "treating" or "treatment" means the treatment (e.g., alleviation or elimination of symptoms and/or cure) and/or prevention or inhibition of the condition (e.g., a skin, mucosal, or nail condition). In one embodiment, the galvanic particulates are administered locally or systemically to the subject (e.g., a human) in need to such treatment. In one embodiment, the galvanic particulates are used to exert their effects on (i.e., to treat, to improve the health of, to cure, to eliminate and/or to reduce the quantity of) a living organism, including vertebrate animals (mammals including human, birds, fish, reptiles, and amphibian), insects, plants, micro-organisms (e.g., bacteria, fungi and viruses).

### Galvanic Particulates

The galvanic particulates of the present invention include a first conductive material and a second conductive material, wherein both the first conductive material and the second conductive material are exposed on the surface of the particulate. In one embodiment, the particulate includes the first conductive material and the surface of the particulate is partially coated with the second conductive material.

In one embodiment, the galvanic particulates are produced by a coating method wherein the weight percentage of the second conductive material is from about 0.001% to about 20%, by weight, of the total weight of the particulate, such as from about 0.01% to about 10%, by weight, of the total weight of the particulate. In one embodiment, the coating thickness of the second conductive material may vary from single atom up to hundreds of microns. In yet another embodiment, the surface of the galvanic particulate comprises from about 0.001 percent to about 99.99 percent such as from about 0.1 to about 99.9 percent of the second conductive material.

In one embodiment, the galvanic particulates are produced by a non-coating method (e.g., by sintering, printing or mechanical processing the first and the second conductive materials together to form the galvanic particulate) wherein the second conductive material comprises from about 0.1% to about 99.9%, by weight, of the total weight of the particulate, such as from about 10% to about 90%, of the total weight of the particulate.

In one embodiment, the galvanic particulates are fine enough that they can be suspended in the semi-solid compositions during storage. In a further embodiment, they are in flattened and/or elongated shapes. The advantages of flattened and elongated shapes of the galvanic particulates include a lower apparent density and, therefore, a better floating/suspending capability in the topical composition, as well as better coverage over the biological tissue, leading to a wider and/or deeper range of the galvanic current passing through the biological tissue (e.g., the skin or mucosa membrane). In one embodiment, the longest dimension of the galvanic particulates is at least twice (e.g., at least five times) the shortest dimension of such particulates.

The galvanic particulates may be of any shape, including but not limited to, spherical or non-spherical particles or elongated or flattened shapes (e.g., cylindrical, fibers or flakes). In one embodiment, the average particle size of the galvanic particulates is from about 10 nanometers to about 500 micrometers, such as from about 100 nanometers to about 100 micrometers. What is meant by the particle size the maximum dimension in at least one direction.

In one embodiment, the galvanic particulate comprises at least 90 percent, by weight, of conductive materials (e.g., the first conductive material and the second conductive material), such as at least 95 percent, by weight, or at least 99 percent, by weight, when a coating method is used for the production of the galvanic particulates.

Examples of combinations of first conductive materials/second conductive materials include (with a "/" sign representing an oxidized but essentially non-soluble form of the metal), but are not limited to, zinc-copper, zinc-copper/copper halide, zinc-copper/copper oxide, magnesium-copper, magnesium-copper/copper halide, zinc-silver, zinc-silver/silver oxide, zinc-silver/silver halide, zinc-silver/silver chloride, zinc-silver/silver bromide, zinc-silver/silver iodide, zinc-silver/silver fluoride, zinc-gold, zinc-carbon, magnesium-gold, magnesium-silver, magnesium-silver/silver oxide, magnesium-silver/silver halide, magnesium-silver/silver chloride, magnesium-silver/silver bromide, magnesium-silver/silver iodide, magnesium-silver/silver fluoride, magnesium-carbon, aluminum-copper, aluminum-gold, aluminum-silver, aluminum-silver/silver oxide, aluminum-silver/silver halide, aluminum-silver/silver chloride, aluminum-silver/silver bromide, aluminum-silver/silver iodide, aluminum-silver/silver fluoride, aluminum-carbon, copper-silver/silver halide, copper-silver/silver chloride, copper-silver/silver bromide, copper-silver/silver iodide, copper-silver/silver fluoride, iron-copper, iron-copper/copper oxide, copper-carbon iron-copper/copper halide, iron-silver, iron-silver/silver oxide, iron-silver/silver halide, iron-silver/silver chloride, iron-silver/silver bromide, iron-silver/silver iodide, iron-silver/silver fluoride, iron-gold, iron-conductive carbon, zinc-conductive carbon, copper-conductive carbon, magnesium-conductive carbon, and aluminum-carbon.

The first conductive material or second conductive material may also be alloys, particularly the first conductive material. Non-limiting examples of the alloys include alloys of zinc, iron, aluminum, magnesium, copper and manganese as the first conductive material and alloys of silver, copper, stainless steel and gold as second conductive material.

In one embodiment, the particulate, made of the first conductive material, is partially coated with several conductive materials, such as with a second and third conductive material. In a further embodiment, the particulate comprises at least 95 percent, by weight, of the first conductive material, the second conductive material, and the third conductive material. In one embodiment, the first conductive material is zinc, the second conductive material is copper, and the third conductive material is silver.

In one embodiment, the difference of the Standard Electrode Potentials (or simply, Standard Potential) of the first conductive material and the second conductive material is at least about 0.1 volts, such as at least 0.2 volts. In one embodiment, the materials that make up the galvanic couple have a standard potential difference equal to or less than about 3 volts. For example, for a galvanic couple comprised of metallic zinc and copper, the Standard Potential of zinc is -0.763V (Zn/Zn2⁺), and the Standard Potential of copper is +0.337 (Cu/Cu2⁻), the difference of the Standard Potential is therefore 1.100V for the zinc-copper galvanic couple. Similarly, for the for the magnesium-copper galvanic couple, Standard Potential of magnesium (Mg/Mg2⁺) is -2.363V, and the difference of the Standard Potential is therefore 2.700V. Additional examples of Standard Potential values of some materials suitable for galvanic couples are: Ag/Ag⁺: +0.799V, Ag/AgCl/Cl⁻: 0.222V, and Pt/H₂/H⁺: 0.000V. Pt may also be replaced by carbon or another conductive material. See, e.g., Physical Chemistry by Gordon M. Barrow, 4th Ed., McGraw-Hill Book Company, 1979, Page 626.

### Manufacture of Galvanic Particulates

In one embodiment, the conductive electrodes are combined (e.g., the second conductive electrode is deposited to the first conductive electrode) by chemical, electrochemical, physical or mechanical process (such as electroless deposition, electric plating, vacuum vapor deposition, arc spray, sintering, compacting, pressing, extrusion, printing, and granulation) conductive metal ink (e.g., with polymeric binders), and other known metal coating and powder processing methods commonly used in powder metallurgy, electronics and medical device manufacturing processes, such as the methods described in the book: "Asm Handbook Volume 7: Powder Metal Technologies and Applications" (by Asm International Handbook Committee, edited by Peter W. Lee, 1998, pages 31-109, 311-320). In another embodiment, all the conductive electrodes are manufactured by chemical reduction processes (e.g., electroless deposition), sequentially or simultaneously, in the presence of reducing agent(s). Examples of reducing agents include phosphorous-containing reducing agents (e.g., a hypophosphite as described in US Patent Nos 4,167,416 and 5,304,403), boron-containing reducing agents, and aldehyde- or keton-containing reducing agents such as sodium tetrahydridoborate (NaBH4) (e.g., as decribed in US 20050175649).

In one embodiment, the second conductive electrode is deposited or coated onto the first conductive electrode by physical deposition, such as spray coating, plasma coating, conductive ink coating, screen printing, dip coating, metals bonding, bombarding particulates under high pressure-high temperature, fluid bed processing, or vacuum deposition.

In one embodiment, the coating method is based on displacement chemical reaction, namely, contacting a particulate of the first conductive material (e.g., metallic zinc particle) with a solution containing a dissolved salt of the second conductive material (e.g. copper acetate, copper lactate, copper gluconate, or silver nitrate). In a further embodiment, the method includes flowing the solution over the particulate of the first conductive material (e.g., zinc powder) or through the packed powder of the first conductive material. In one embodiment, the salt solution is an aqueous solution. In another embodiment, the solution is contains an organic solvent, such as an alcohol, a glycol, glycerin or other commonly used solvents in pharmaceutical production to regulate the deposition rate of the second conductive material onto the surfaces of the first particulates, therefore controlling the activity of the galvanic particulates produced.

In another embodiment, the galvanic particulates of the present invention may also be coated with other materials to protect the galvanic materials from degradation during storage (e.g, oxidation degradation from oxygen and moisture), or to modulate the electrochemical reactions and to control the electric current generate when in use. The exemplary coating materials over the galvanic material(s) are inorganic or organic polymers, natural or synthetic polymers, biodegradable or bioabsorbable polymers, silica, glass, various various metal oxides (e.g, oxide of zinc, aluminum, magnisum, or titanium) and other inorganic salts of low solubility (e.g, zinc phosphate). The coating methods are known in the art of metallic powder processing and metal pigment productions, such as those decribed by U.S. Patent publicatons US 5,964,936; U.S. 5,993,526; US 7,172812; US 20060042509A1 and US 20070172438.

In one embodiment, the galvanic particulates are stored in anhydrous forms, e.g., as a dry powder or immobilized in a fabric with binding agents, or as an essentially anhydrous non-conducting organic solvent composition (e.g., dissolved in polyethylene glycols, propylene glycol, glycerin, liquid silicone, and/or alcohol). In another embodiment, the galvanic particulates are embedded into the anhydrous carrier (e.g., inside a polymer) or coated onto a substrate (e.g., as a coating or in the coating layer of a healthcare product such as wound dressing or dental floss). In yet another embodiment, the galvanic particulates are encapsulated in compositions of microcapsules, liposomes, micelles, or embedded in the lipophilic phase of oil-in-water (O/W) or water-in-oil (W/O) types of emulsion systems (e.g., W/O lotion, W/O ointment, or O/W creams), as well as self-emulsifying compositions, in order to achieve self-life stability, retard the activation of the galvanic particulates, or prolong the action of galvanic particulates.

The galvanic particulates may also be compressed into tablets, incorporated into the polymer composition in the tablet coating film, incorporated into either hard or soft gelatin capsules, or incorporated waxy materials (e.g., as used in suppositories) or polymers (into bioabsorbable polymers as used in implant products or into biocompatible polymers as used in dental bracelets and toothbrushes). The coating (shell) materials used in the microcapsules may have an enteric property (e.g., being insoluble at acidic condition and only soluble when exposed to a medium with the pH value near or equal to neutral pH), or have a pH-sensitive permeability for the water and solute molecules and ions, or is biodegradable or bioabsorbable.

### Compositions and Products

The galvanic particulates have great versatility in applications, and can be used in many consumer and medical products for human and animal applications such as ingestible compositions (such as tablets and solutions), topical compositions (such as creams, lotions, gels, shampoos, cleansers, powders patches, bandages, and masks for application to the skin or mucosal membranes), garments (such as undergarments, underwears, bras, shirts, pants, pantyhose, socks, head caps, facial masks, gloves, and mittens), linens (such as towels, pillow covers or cases and bed sheets), and personal and medical products (such as sanitizing products for household and clinical settings, microcides for plants) and devices (such as toothbrushes, dental flosses, periodontal implants or inserts, orthodontic braces, joint wraps/supports, buccal patches, ocular inserts or implants such as contact lenses, nasal implants or inserts, and contact lens cleaning products, wound dressings, diapers, sanitary napkins, and wipes, tampons, rectal and vaginal suppositories, and galvanic particulates-coatings or -embedded surfaces on the medical devices and other surfaces where the antimicrobial or other beneficial effects are desired). Many of such compositions and products are further discussed below.

In one embodiment, the galvanic particulates induce certain desirable biological responses that facilitate the treatment of the barrier membrane conditions (e.g., induced by the electric current passage through the skin, intestine, or mucosal membrane and/or enhancing the delivery of an active agent). In one embodiment, the galvanic particulates provide multiple mechanism of actions to treat conditions, such as to enhance delivery of an active agents by iontophoresis and/or electro-osmosis as well as provide electric stimulation to treat the contacted tissue (e.g., to increase blood circulation or other benefits).

What is meant by an "active agent" is a compound (e.g., a synthetic compound or a compound isolated from a natural source) that has a cosmetic or therapeutic effect on the barrier membrane and the surrounding tissues (e.g., a material capable of exerting a biological effect on a human body) such as therapeutic drugs or cosmetic agents. Examples of such therapeutic drugs include small molecules, peptides, proteins, nucleic acid materials, and nutrients such as minerals and extracts. The amount of the active agent in the carrier will depend on the active agent and/or the intended use of the composition or product. In one embodiment, the composition containing the galvanic particulates further contain a safe and effective amount of the active agent, for example, from about 0.001 percent to about 20 percent, by weight, such as from about 0.01 percent to about 10 percent, by weight, of the composition.

The galvanic particulates can be combined with an active agent (such as antimicrobial agents, anti-inflammatory agents, and analgesic agents) to enhance or potentiate the biological or therapeutic effects of that active agent. In another embodiment, the galvanic particulates can also be combined with other substances to enhance or potentiate the activity of the galvanic particulates. Substances that can enhance or potentiate the activity of the galvanic particulates include, but are not limited to, organic solvents (such as alcohols, glycols, glycerin, polyethylene glycols and polypropylene glycol), surface active agents (such as nonionic surfactants, zwitterionic surfactants, anionic surfactants, cationic surfactants and polymeric surfactants), and water-soluble polymers. For example, the galvanic particulates of the present invention can form conjugates or composites with synthetic or natural polymers including by not limited to proteins, polysaccharides, hyaluronic acid of various molecular weight, hyaluronic acid analogs, polypeptides, and polyethylene glycols.

In one embodiment, the composition contains a chelator or chelating agent. Examples of chelators include, but are not limited to, amino acids such as glycine, lactoferrin, edetate, citrate, pentetate, tromethamine, sorbate, ascorbate, deferoxamine, derivatives thereof, and mixtures thereof. Other examples of chelators useful are disclosed in U.S. Pat. No. 5,487,884 and PCT Publication Nos. 91/16035 and 91/16034.

### Methods of Using Galvanic Particulates

In one embodiment, the galvanic particulates are used to provide the intended therapeutic electric stimulation effects by applying the galvanic particulates directly to the target location of the body in need such a therapeutic treatment (e.g., either topically or inside the body), including soft tissues (e.g., the skin, mucosa, epithelium, wound, eye and its surrounding tissues, cartilage and other soft musculoskeletal tissues such as ligaments, tendons, or meniscus), hard tissues (e.g., bone, teeth, nail matrix, or hair follicle), and soft tissue-hard tissue conjunctions (e.g., conductive tissues around periodontal area involved teeth, bones or soft tissue of the joint).

Such therapeutic effects include, but are not limited to: antimicrobial effects (e.g., antibacterial, antifungal, antiviral, and anti-parasitic effects); anti-inflammation effects including effects in the superficial or deep tissues (e.g., reduce or elimination of soft tissue edema or redness); elimination or reduction of pain, itch or other sensory discomfort (e.g., headache, sting or tingling numbness); regeneration or healing enhancement of both soft and hard tissues; modulation of stem cell differentiation and tissue development such as modulation of tissue growth (e.g., enhancing growth rate of the nail or regrowth of hair loss due to alopecia) or increase soft tissue volume (e.g., increasing collagen or elastin in the skin or lips); increasing adepocyte metabolism or improving body appearance (e.g., effects on body contour or shape); and increasing circulation of blood or lymphocytes.

One skilled in the art will recognize that, both *in vivo* and *in vitro* trials using suitable, known and generally accepted cell and / or animal models are predictive of the ability of an ingredient, composition, or product to treat or prevent a given condition. One skilled in the art will further recognize that human clinical trails including first-in-human, dose ranging and efficacy trials, in healthy patients and / or those suffering from a given condition or disorder, may be completed according to methods well known in the clinical and medical arts.

### Ingestible Compositions

The ingestible compositions useful in the present invention involve compositions suitable for ingesting by the mammal, such as a human, in need to such treatment. In one embodiment, the compositions contain a safe and effective amount of (i) the galvanic particulates and (ii) a pharmaceutically-acceptable carrier.

In one embodiment, the ingestible compositions herein contain, per dosage unit (e.g., tablet, capsule, powder, injection, teaspoonful and the like) an amount of the galvanic particulates and/or active agent necessary to deliver an effective dose as described above. In one embodiment, the ingestible compositions herein contains, per unit dosage unit of from about 1 mg to about 5 g of the galvanic particulates and/or active agent, such as from about 50 mg to about 500 mg, and may be given at a dosage of from about 1 mg/kg/day to about 1 g/kg/day, such as from about 50 to about 500 mg/kg/day. The dosages, however, may be varied depending upon the requirement of the patients, the severity of the condition being treated, and the galvanic particulates being employed. The use of either daily administration or post-periodic dosing may be employed. In one embodiment, these compositions are in unit dosage forms from such as tablets, pills, capsules, powders, granules, solutions or suspensions, and drops.

In one embodiment, the compositions are provided in the form of tablets, such as those containing 1, 5, 10, 25, 50, 100, 150, 200, 250, 500, and/or 1000 milligrams of the galvanic particulates and/or active agent for the symptomatic adjustment of the dosage to the patient to be treated. The composition may be administered on a regimen of 1 to 4 times per day. Advantageously, the compositions may be administered in a single daily dose, or the total daily dosage may be administered in divided doses of two, three or four times daily.

Optimal dosages to be administered may be readily determined by those skilled in the art, and will vary with the particular galvanic particulates and/or active agent used, the mode of administration, the strength of the preparation, and the advancement of the disease/ condition being treated. In addition, factors associated with the particular patient being treated, including patient age, weight, diet and time of administration, will result in the need to adjust dosages.

Ingestible compositions containing one or more types of the galvanic particulates of the invention described herein can be prepared by intimately mixing the galvanic particulates with a pharmaceutically-acceptable carrier according to conventional pharmaceutical compounding techniques. The carrier may take a wide variety of forms depending upon the type of formulation. Thus for liquid preparations such as suspensions, elixirs and solutions, suitable carriers and additives include but not limited to water, glycols, alcohols, silicones, waxes, flavoring agents, buffers (such as citrate buffer, phosphate buffer, lactate buffer, gluconate buffer), preservatives, stabilizers, coloring agents and the like; and for solid preparations, such as powders, capsules and tablets, suitable carriers and additives include starches, sugars, diluents, granulating agents, lubricants, binders, disintegrating agents and the like. Solid oral preparations may also be coated with substances such as sugars, soluble polymer film, insoluble-but-solute permeable polymer film. Oral preparation may also be coated with enteric coating, which is not soluble in the acidic stomach environment but will dissolve in the intestine as the pH becomes neutral so as to modulate major site of galvanic particulate action. For product storage and stability, the galvanic particulates should preferably be kept in an anhydrous or relatively non-conductive phase or compartment.

For preparing solid compositions such as tablets, the galvanic particulates are mixed with a pharmaceutically-acceptable carrier, e.g. conventional tableting ingredients such as corn starch, lactose, sucrose, sorbitol, talc, stearic acid, magnesium stearate, dicalcium phosphate or gums, and other pharmaceutically-acceptable diluents, to form a solid preformulation composition containing a homogeneous mixture of the galvanic particulates. When referring to these preformulation compositions as homogeneous, it is meant that the galvanic particulates is dispersed evenly throughout the composition so that the composition may be readily subdivided into equally effective dosage forms such as tablets, pills and capsules. This solid preformulation composition may then subdivided into unit dosage forms of the type described above. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

### (a) Gastro-intestinal Disorder Treatment Ingestible Compositions

In one embodiment, ingestible compositions containing the galvanic particulates are used for the treatment of gastrointestinal disorders, such as ulcers, diarrhea, and gastrointestinal pain.

In one embodiment, the galvanic particulates can be combined with active agents known to treat diarrhea which include, but are not limited to: bismuths (such as Bismuth Subsalicylate), Loperamide, Simethicone, Nitazoxanide, Ciprofloxacin, and Rifaximin, salts and prodrugs (such as esters) thereof.

In one embodiment, the galvanic particulates can be combined with active agents known to treat gastric ulcers which include, but are not limited to: Lansoprazole, Naproxen, Esomeprazole, Famotidine, Nizatidine, Ranitidine, and Omeprazole, and salts and prodrugs thereof.

In one embodiment, the galvanic particulates can be combined with active agents known to treat intra-abdominal infections which include, but are not limited to: Moxifloxacin, Ciprofloxacin, Ceftazidime, Gentamicin, Ertapenem; Cefepime, Cefoxitin, Cilastatin, Imipenem; Ceftriaxone, Clavulanate, and Ticarcillin, and salts and prodrugs thereof.

### (b) Pain Treating Ingestible Compositions

In one embodiment, ingestible compositions containing the galvanic particulates are used for treatment of pain (such as throat pain). Oral dosage forms can be in the forms of, but not limited to, lozenges or liquids. Galvanic particulates can be combined with active agents known to treat sore throat, which include, but are not limited to: Acetaminophen, Dextromethorphan, Pseudoephedrine, Chlorpheniramine, Pseudoephedrine, Guaifenesin, Doxylamine, Zinc, and Ibuprofen, and salts and prodrugs thereof.

### (c) Oral Supplement Ingestible Compositions

In one embodiment, ingestible compositions containing the galvanic particulates are used as oral supplements or complements to oral supplements. Oral dosage forms can be in the forms of, but not limited to, lozenges, tablets, caplets, powders, or liquids. Galvanic particulates can be combined with oral supplements of vitamins and minerals, which include, but are not limited to: Dibasic Calcium Phosphate, Magnesium Oxide, Potassium Chloride, Microcrystalline Cellulose, Ascorbic Acid (Vit. C), Ferrous Fumarate, Calcium Carbonate, dl-Alpha Tocopheryl Acetate (Vit. E), Acacia, Ascorbyl Palmitate, Beta Carotene, Biotin, BHT, Calcium Pantothenate, Calcium Stearate, Chromic Chloride, Citric Acid, Crospovidone, Cupric Oxide, Cyanocobalamin (Vit. B ₁₂), Ergocalciferol (Vit. D), Folic Acid, Gelatin, Hypromellose, Lutein, Lycopene, Magnesium Borate, Magnesium Stearate, Manganese Sulfate, Niacinamide, Nickelous Sulfate, Phytonadione (Vit. K), Potassium Iodide, Pyridoxine Hydrochloride (Vit. B ₆), Riboflavin (Vit. B ₂), Silicon Dioxide, Sodium Aluminum Silicate, Sodium Ascorbate, Sodium Benzoate, Sodium Borate, Sodium Citrate, Sodium Metavanadate, Sodium Molybdate, Sodium Selenate, Sorbic Acid, Stannous Chloride, Sucrose, Thiamine Mononitrate (Vit. B ₁ ), Titanium Dioxide, Tribasic Calcium Phosphate, Vitamin A Acetate (Vit. A), and Zinc Oxide., and salts and prodrugs thereof

In addition, in one embodiment, the metal components of the galvanic particulates can serve as mineral supplements generated in situ, e.g. zinc metal converted to zinc ion in situ.

### Topical Skin Compositions

In one embodiment, topical compositions useful in the present invention involve compositions containing the galvanic particulates that are suitable for administering to mammalian skin, such as human skin. In one embodiment, the compositions contain a safe and effective amount of (i) the galvanic particulates and (ii) a pharmaceutically-acceptable carrier.

The compositions may be made into a wide variety of products that include but are not limited to leave-on products (such as lotions, creams, gels, sticks, sprays, and ointments), skin cleansing products (such as liquid washes, solid bars, and wipes), hair products (such as shampoos, conditioners, sprays, and mousses), shaving creams, film-forming products (such as masks), make-up (such as foundations, eye liners, and eye shadows), deodorant and antiperspirant compositions, and the like. These product types may contain several types of pharmaceutically-acceptable carrier forms including, but not limited to solutions, suspensions, emulsions such as microemulsions and nanoemulsions, gels, and solids carrier forms. Other product forms can be formulated by those of ordinary skill in the art.

In one embodiment, the composition or product is used for the treatment of skin conditions. Examples of such treatments include, but are not limited to: the treatment of acne (e.g., blackheads and whiteheads), rosacea, nodule-cystic, and other microbial infections of the skin; reduction the visible signs of skin aging (e.g., wrinkles, sagging, sallowness, and age-spots); firming the skin; enhancing the elasticity of the skin; folliculitis and pseudo-folliculitis barbae; sebum regulation (e.g., sebum reduction or oily/shining skin appearance inhibition or control); pigmentation regulation (e.g., reduction of hyperpigmentation such as freckles, melasma, actinic and senile lentigines, age-spots, post-inflammatory hypermelanosis, Becker's naevus, and facial melanosis or enhancing the pigmentation of light skin); hair growth retardation (e.g., skin on the leg) or hair stimulation (e.g., to the scalp); and the treatment of dermatitis (e.g., atopic, contact, or seborrheic dermatitis), dark circles under the eye, stretch marks, cellulite, excessive sweating (e.g., hyperhidrosis), and/or psoriasis.

### (a) Topical Anti-acne/Anti-rosacea Compositions

In one embodiment, the composition or product contains an anti-acne and/or anti-rosacea active agent. Examples of anti-acne and anti-rosacea agents include, but are not limited to: retinoids such as tretinoin, isotretinoin, motretinide, adapalene, tazarotene, azelaic acid, and retinol; salicylic acid; benzoyl peroxide; resorcinol; sulfur; sulfacetamide; urea; antibiotics such as tetracycline, clindamycin, metronidazole, and erythromycin; anti-inflammatory agents such as corticosteroids (e.g., hydrocortisone), ibuprofen, naproxen, and hetprofen; and imidazoles such as ketoconazole and elubiol; and salts and prodrugs thereof. Other examples of anti-acne active agents include essential oils, alpha-bisabolol, dipotassium glycyrrhizinate, camphor, β-glucan, allantoin, feverfew, flavonoids such as soy isoflavones, saw palmetto, chelating agents such as EDTA, lipase inhibitors such as silver and copper ions, hydrolyzed vegetable proteins, inorganic ions of chloride, iodide, fluoride, and their nonionic derivatives chlorine, iodine, fluorine, and synthetic phospholipids and natural phospholipids such as Arlasilk™ phospholipids CDM, SV, EFA, PLN, and GLA (Uniqema, ICI Group of Companies, Wilton, UK).

### (b) Topical Anti-aging Compositions

In one embodiment, the composition or product contains an anti-aging agent. Examples of suitable anti-aging agents include, but are not limited to: inorganic sunscreens such as titanium dioxide and zinc oxide; organic sunscreens such as octyl-methoxy cinnamates; retinoids; dimethylaminoathanol (DMAE), copper containing peptides, vitamins such as vitamin E, vitamin A, vitamin C, and vitamin B and vitamin salts or derivatives such as ascorbic acid di-glucoside and vitamin E acetate or palmitate; alpha hydroxy acids and their precursors such as glycolic acid, citric acid, lactic acid, malic acid, mandelic acid, ascorbic acid, alpha-hydroxybutyric acid, alpha- hydroxyisobutyric acid, alpha-hydroxyisocaproic acid, atrrolactic acid, alpha-hydroxyisovaleric acid, ethyl pyruvate, galacturonic acid, glucoheptonic acid, glucoheptono 1,4-lactone, gluconic acid, gluconolactone, glucuronic acid, glucuronolactone, isopropyl pyruvate, methyl pyruvate, mucic acid, pyruvic acid, saccharic acid, saccaric acid 1,4-lactone, tartaric acid, and tartronic acid; beta hydroxy acids such as beta-hydroxybutyric acid, beta-phenyl-lactic acid, and beta-phenylpyruvic acid; tetrahydroxypropyl ethylene-diamine, N,N,N',N'-Tetrakis(2-hydroxypropyl)ethylenediamine (THPED); and botanical extracts such as green tea, soy, milk thistle, algae, aloe, angelica, bitter orange, coffee, goldthread, grapefruit, hoellen, honeysuckle, Job's tears, lithospermum, mulberry, peony, puerarua, nice, and safflower; and salts and prodrugs thereof.

### (c) Topical Depigmentation Compositions

In one embodiment, the composition or product contains a depigmentation agent. Examples of suitable depigmentation agents include, but are not limited to: soy extract; soy isoflavones; retinoids such as retinol; kojic acid; kojic dipalmitate; hydroquinone; arbutin; transexamic acid; vitamins such as niacin and vitamin C; azelaic acid; linolenic acid and linoleic acid; placertia; licorice; and extracts such as chamomile and green tea; and salts and prodrugs thereof.

### (d) Topical Antipsoriatic Compositions

In one embodiment, the composition or product contains an antipsoriatic active agent. Examples of antipsoriatic active agents (e.g., for seborrheic dermatitis treatment) include, but are not limited to, corticosteroids (e.g., betamethasone dipropionate, betamethasone valerate, clobetasol propionate, diflorasone diacetate, halobetasol propionate, triamcinonide, dexamethasone, fluocinonide, fluocinolone acetonide, halcinonide, triamcinolone acetate, hydrocortisone, hydrocortisone verlerate, hydrocortisone butyrate, aclometasone dipropionte, flurandrenolide, mometasone furoate, methylprednisolone acetate), methotrexate, cyclosporine, calcipotriene, anthraline, shale oil and derivatives thereof, elubiol, ketoconazole, coal tar, salicylic acid, zinc pyrithione, selenium sulfide, hydrocortisone, sulfur, menthol, and pramoxine hydrochloride, and salts and prodrugs thereof.

### (e) Other Ingredients

In one embodiment, the composition or product contains a plant extract as an active agent. Examples of plant extracts include, but are not limited to, feverfew, soy, glycine soja, oatmeal, what, aloe vera, cranberry, witch-hazel, alnus, arnica, artemisia capillaris, asiasarum root, birch, calendula, chamomile, cnidium, comfrey, fennel, galla rhois, hawthorn, houttuynia, hypericum, jujube, kiwi, licorice, magnolia, olive, peppermint, philodendron, salvia, sasa albo- marginata, natural isoflavonoids, soy isoflavones, and natural essential oils.

In one embodiment, the composition or product contains a buffering agent such as citrate buffer, phosphate buffer, lactate buffer, gluconate buffer, or gelling agents, thickeners, or polymers.

In one embodiment, the composition or product contains a fragrance effective for reducing stress, calming, and/or affecting sleep such as lavender and chamomile.

### Topical Mucosal Compositions

In one embodiment, topical compositions useful in the present invention involve compositions containing the galvanic particulates that are suitable for administering to the mucosal membrane, such as human oral, rectal, and vaginal musocal membranes. In one embodiment, the compositions contain a safe and effective amount of (i) the galvanic particulates and (ii) a pharmaceutically-acceptable carrier.

The compositions may be made into a wide variety of products for application on mucosa, including but not limited to vaginal creams, tampons, suppositories, floss, mouthwash, toothpaste. Other product forms can be formulated by those of ordinary skill in the art.

In one embodiment, the composition or product is used for the treatment of a mucosal membrane conditions. Examples of such treatments include, but are not limited to, treatment of vaginal candidiasis and vaginosis, genital and oral herpes, cold sore, canker sore, oral hygiene, periodontal disease, teeth whitening, halitosis, prevention of biofilm attachment, and other microbial infections of the mucosa.

The galvanic particulates can be incorporated into compositions for the treatment of candidiasis with actives such as, but not limited to: Tioconazole; Clotrimazole; and Nystatin.

The galvanic particulates can be incorporated into compositions for the treatment of bacterial vaginosis with actives such as, but not limited to, Clindamycin Hydrochloride and Metronidazole.

The galvanic particulates can be incorporated into compositions for the treatment of periodontal disease with actives such as, but not limited to minocycline.

### Compositions for Treatment of Wounds and Scars

In one embodiment, the galvanic particulates are incorporated into wound dressings and bandages to provide electric therapy for healing enhancement and scar prevention. In one embodiment, the wound exudation fluid and/or wound cleansing solution serves to activate a galvanic particulate containing wound dressing/bandage to (i) deliver active agents preincorporated in the wound dressing/bandage and/or (ii) to generate electrochemically beneficial metal ions followed with delivery of the beneficial metal ions into the wound and/or (iii) treat the wound with therapeutic electric current which may increase blood circulation, stimulate tissue immune response, and/or suppress tissue inflammation, which may lead to accelerated healing and reduced scarring.

In one embodiment, the composition or product contains an active agent commonly used as for topical wound and scar treatment, such as topical antibiotics, anti-microbials, wound healing enhancing agents, topical antifungal drugs, anti-psoriatic drugs, and antiinflammatory agents.

Examples of antifungal drugs include but are not limited to miconazole, econazole, ketoconazole, sertaconazole, itraconazole, fluconazole, voriconazole, clioquinol, bifoconazole, terconazole, butoconazole, tioconazole, oxiconazole, sulconazole, saperconazole, clotrimazole, undecylenic acid, haloprogin, butenafine, tolnaftate, nystatin, ciclopirox olamine, terbinafine, amorolfine, naftifine, elubiol, griseofulvin, and their pharmaceutically acceptable salts and prodrugs. In one embodiment, the antifungal drug is an azole, an allylamine, or a mixture thereof.

Examples of antibiotics (or antiseptics) include but are not limited to mupirocin, neomycin sulfate bacitracin, polymyxin B, 1-ofloxacin, tetracyclines (chlortetracycline hydrochloride, oxytetracycline - 10 hydrochloride and tetrachcycline hydrochoride), clindamycin phsphate, gentamicin sulfate, metronidazole, hexylresorcinol, methylbenzethonium chloride, phenol, quaternary ammonium compounds, tea tree oil, and their pharmaceutically acceptable salts and prodrugs.

Examples of antimicrobials include but are not limited to salts of chlorhexidine, such as lodopropynyl butylcarbamate, diazolidinyl urea, chlorhexidene digluconate, chlorhexidene acetate, chlorhexidene isethionate, and chlorhexidene hydrochloride. Other cationic antimicrobials may also be used, such as benzalkonium chloride, benzethonium chloride, triclocarbon, polyhexamethylene biguanide, cetylpyridium chloride, methyl and benzothonium chloride. Other antimicrobials include, but are not limited to: halogenated phenolic compounds, such as 2,4,4',-trichloro-2- hydroxy diphenyl ether (Triclosan); parachlorometa xylenol (PCMX); and short chain alcohols, such as ethanol, propanol, and the like. In one embodiment, the alcohol is at a low concentration (e.g., less than about 10% by weight of the carrier, such as less than 5% by weight of the carrier) so that it does not cause undue drying of the barrier membrane.

Examples of anti-viral agents for viral infections such as herpes and hepatitis, include, but are not limited to, imiquimod and its derivatives, podofilox, podophyllin, interferon alpha, acyclovir, famcyclovir, valcyclovir, reticulos and cidofovir, and salts and prodrugs thereof.

Examples of anti-inflammatory agent, include, but are not limited to, suitable steroidal anti-inflammatory agents such as corticosteroids such as hydrocortisone, hydroxyltriamcinolone alphamethyl dexamethasone, dexamethasone-phosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclarolone acetonide, fludrocortisone, flumethasone pivalate, fluosinolone acetonide, fluocinonide, flucortine butylester, fluocortolone, fluprednidene (fluprednylidene)acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenalone acetonide, medrysone, amciafel, amcinafide, betamethasone, chlorprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylproprionate, hydrocortamate, meprednisone, paramethasone, prednisolone, prednisone, beclomethasone dipropionate, betamethasone dipropionate, triamcinolone, and salts are prodrugs thereof. In one embodiment, the steroidal anti-inflammatory for use in the present invention is hydrocortisone. A second class of anti-inflammatory agents which is useful in the compositions of the present invention includes the nonsteroidal anti-inflammatory agents.

Examples of wound healing enhancing agent include recombinant human platelet-derived growth factor (PDGF) and other growth factors, ketanserin, iloprost, prostaglandin E₁ and hyaluronic acid, scar reducing agents such as mannose-6-phosphate, analgesic agents, anesthetics, hair growth enhancing agents such as minoxadil, hair growth retarding agents such as eflornithine hydrochloride, antihypertensives, drugs to treat coronary artery diseases, anticancer agents, endocrine and metabolic medication, neurologic medications, medication for cessation of chemical additions, motion sickness, protein and peptide drugs.

### Treatment of Microbial Infections of the Body

In one embodiment, the galvanic particulates are used, with or without other antifungal active agents, to treat and prevent fungal infections (e.g., dermatophytes such as trichophyton mentagrophytes), including, but not limited to, onychomycosis, sporotrichosis, tinea unguium, tinea pedis (athlete's foot), Tinea cruris (jock itch), tinea corporis (ringworm), tinea capitis, tinea versicolor, and candida yeast infection-related diseases (e.g., candida albicans) such as diaper rash, oral thrushm, cutaneous and vaginal candidiasis, genital rashes, Malassezia furfur infection- related diseases such as Pityriasis versicolor, Pityriasis folliculitis, seborrhoeic dermatitis, and dandruff.

In another embodiment, the galvanic particulates are used, with or without other antibacterial active agents, to treat and prevent bacterial infections, including, but not limited to, acne, cellulitis, erysipelas, impetigo, folliculitis, and furuncles and carbuncles, as well as acute wounds and chronic wounds (venous ulcers, diabetic ulcers and pressure ulcers).

In another embodiment, the galvanic particulates are used, with or without other antiviral active agents, to treat and prevent viral infections of the skin and mucosa, including, but not limited to, molluscum contagiosum, warts, herpes simplex virus infections such as cold sores, kanker sores and genital herpes.

In another embodiment, the galvanic particulates are used, with or without other antiparasitic active agents, to treat and prevent parasitic infections, including, but not limited to, hookworm infection, lice, scabies, sea bathers' eruption and swimmer's itch.

In one embodiment, the particulates are administered to help treat ear infections (such as those caused by streptococcus oneumoniae), rhinitis and/or sinusitis (such as caused by Haemophilus influenzae, Moraxella catarrhalis, Staphylococcus aureus and Streptococcus pneumoniae), and strep throat (such as caused by Streptococcus pyogenes).

In one embodiment, the particulates are ingested by an animal (e.g., as animal feed) or a human (e.g., as a dietary supplement) to help prevent outbreaks of food borne illnesses (e.g., stemming from food borne pathogens such as Campylobacter jejuni, Listeria monocytogenes, and Salmonella enterica).

### Drug Resistant Microorganisms

In one embodiment, the invention features a method of killing pathogens drug resistant microorganisms by contacting the microorganism with a composition containing a galvanic particulate including a first conductive material and a second conductive material, wherein both the first conductive material and the second conductive material are exposed on the surface of the particulate, and wherein the difference of the standard potentials of the first conductive material and the second conductive material is at least about 0.2 V. In one embodiment, the particle size of said particulate is from about 10 nanometers to about 1000 micrometers, such as from about 1 micrometer to about 100 micrometers. In one embodiment, the second conductive material is from about 0.01 percent to about 10 percent, by weight, of the total weight of the particulate. In one embodiment, the drug resistant microoriganism is a bacteria, such as MRSA and VRE. In one embodiment, the particulates are administered via a nasal spray, rinse solution, or ointment.

### Nail Treatment Composition

The galvanic particulates can also be used to stimulate nail growth, enhance nail strength, and reduce nail infection or discoloration. The galvanic particulates can be incorporated into compositions for the treatment of onychomychosis with actives such as, but not limited to: miconazole, econazole, ketoconazole, sertaconazole, itraconazole, fluconazole, voricoriazole, clioquinol, bifoconazole, terconazole, butoconazole, tioconazole, oxiconazole, sulconazole, saperconazole, clotrimazole, undecylenic acid, haloprogin, butenafine, tolnaftate, nystatin, ciclopirox olamine, terbinafine, amorolfine, naftifine, elubiol, griseofulvin, and their pharmaceutically acceptable salts and prodrugs. Galvanic particulates can be incorporated into compositions for improving the look and feel of nails with ingredients such as, but not limited to: biotin, calcium panthotenate, tocopheryl acetate, panthenol, phytantriol, cholecalciferol, calcium chloride, Aloe Barbadensis (Leaf Juice), silk Protein, soy protein, hydrogen peroxide, carbamide peroxide, green tea extract, acetylcysteine and cysteine.

### Tissue-augmentation Composition

In one embodiment, the galvanic particulates can be used to reduce the visibility of skin facial wrinkles, reduce atrophy, or increase collagen stimulation. The galvanic particulates may be used either alone or in conjunction with other components well known in the art, such as subcutaneous fillers, implants, periodontal implants, intramuscular injections, and subcutaneous injections, such as bio-absorbable polymers. For example, the galvanic particulates may be used in conjunction with collagen and/or hyaluronic acid injections.

In another embodiment, the galvanic particulates can be incorporated into biodegradable scaffolds for tissue engineering and organ printing with techniques known in the art.

### Transdermal Drug Delivery Patches

In one embodiment, the galvanic particulates are incorporated into transdermal drug delivery patches to enhance active agent penetration into the skin by iontophoresis and to reduce skin irritation by electric stimulation and electrically generated beneficial ions, such as zinc ions.

Examples of such active agents include peptides, polypeptides, proteins, and nucleic acid materials comprising DNA; and nutrients. Examples of polypeptide and protein active agents include thyrotropin-releasing hormone (TRH), vasopressin, gonadotropin-releasing hormone (GnRH or LHRH), melanotropin-stimulating hormone (MSH), calcitonin, growth hormone releasing factor (GRF), insulin, erythropoietin (EPO), interferon alpha, interferon beta, oxytocin, captopril, bradykinin, atriopeptin, cholecystokinin, endorphins, nerve growth factor, melanocyte inhibitor-1, gastrin antagonist, somatotatin, encephalins, melatonin, vaccines, botox (Botulinum neurotoxins), cyclosporin and its derivatives (e.g., biologically active fragments or analogs). Other active agents include anesthetics; analgesics (e.g., fentanyl and salts thereof such fentanyl citrate); drugs for treating psychiatric disorders, epilepsies, and migraine; drugs for stopping drug additions and abuses; anti-inflammatory agents; drugs to treat hypertension, cardiovascular diseases, gastric acidity and ulcers; drugs for hormone replacement therapies and contraceptives such as estrogens and androgens; antibiotics, antifungals, antiviral and other antimicrobial agents; antineoplastic agents, immunosuppressive agents and immunostimulants; and drugs acting on blood and the blood forming argans including hematopoietic agents and anticoagulants, thrombolytics, and antiplatelet drugs. Other active agents that can be delivered into the body using such patches include vaccines for various diseases, such as those for influenza, AIDS, hepatitis, measles, mumps, rubella, rabies, rubella, avercella, tetanus, hypogammaglobulinemia, Rh disease, diphtheria, botulism, snakebite, back widow bite and other insect bite/sting, idiopathic thrombocytopenic purpura (ITP), chronic lymphocytic leukemia, cytomegalovirus (CMV) infection, acute renal rejection, oral polio, tuberculosis, pertussis, Haemophilus b, Pneumococcus, and *Staphylococcus aureus.*

### Incorporation onto Substrates

The galvanic particulates can be incorporated onto fibers, nonwovens, hydrocolloids, adhesives, films, polymers, and other substrates. Products include but are not limited to dental floss, toothbrushes, sanitary napkins, tampons, bandages, wound dressings, casts, hairbrushes, and clothing. In one embodiment, the galvanic particulates are in contact with the tissue interface. Methods of applying the galvanic particulates on the substrates include electrostatic spray coating, mechanical sieving, co-extrusion, adhesive spraying,

The partilciates may also be coated onto medical implants or surgical tools (e.g., to help prevent infections).

### Examples

The present invention will be further illustrated below by way of Examples, but the present invention is not limited thereto.

### Example 1- Galvanic Particulate Preparation Based on Displacement Chemistry

(a) In Pure Aqueous Media: 0.1% copper coated zinc galvanic particulates were manufactured by electroless plating of copper onto zinc powder. 10g of ≤45-micron zinc powder was spread evenly onto a vacuum filter buchner funnel with a 0.22 micron filter. 5g of copper acetate solution was then poured evenly onto the zinc powder, and allowed to react for approximately 30 seconds. A suction was then applied to the filter until the filtrate was completely suctioned out. The resulting powder cake was then loosed, and 10 g of deionized water was added and then suctioned off. 10g of ethanol was then added to the powder under suction. The powder was then carefully removed from the filter system and allowed to dry in a desiccator.
(b) In Ethanol Containing Media: 0.1% copper coated zinc galvanic particulates were manufactured by electroless plating of copper onto zinc powder. 10g of ≤45-micron zinc powder was weighed into a glass jar. 0.61% w/w copper acetate was dissolved into 200 proof ethanol. The resulting copper solution is a faint blue color. 5g of copper acetate solution was then poured evenly onto the zinc powder, and allowed to react until the copper solution became clear. This reaction continued for approximately 48 hours at room temperature, when the solution turned clear. The composite was spread evenly onto a vacuum filter buchner funnel with a 0.22 micron filter. Vacuum suction was then applied to the filter until the filtrate was completely suctioned out. The resulting powder cake was then loosed, and 10 g of deionized water was added and then suctioned off. 10g of ethanol was then added to the powder under suction. The powder was then carefully removed from the filter system and allowed to dry in a desiccator.
(c) In Pure Aqueous Media: Approximately 0.1% copper coated magnesium galvanic particulates were manufactured by electroless plating of copper onto magnesium powder using the same method described in the Example 1 (a), except substituting zinc powder with magnesium powder.
(d) In Pure Aqueous Media: Approximately 0.1% iron coated magnesium galvanic particulates were manufactured by electroless plating of iron onto magnesium powder using same method described in the Example 1 (a), except substituting zinc powder with magnesium powder and the copper lactate solution with a ferrous chloride solution.

### Example 2 - Coating Galvanic Particulates onto hydrocolloid substrate

(a) Coating Process by Powder Sieving Deposition Onto a Substrate: First, the surface area of the self-adhesive hydrocolloid was measured and the amount of required galvanic particulates was calculated based on a 1.2 mg/cm² surface coating. The galvanic particulates of Example 1(a) were placed into a sieve #325 (45 micron) with the hydrocolloid sheet placed below the sieve. The sieve was gently shaken to produce an even coating of powders onto the hydrocolloid surface. A PET release liner was placed onto the galvanic particulate-coated hydrocolloid surface. The release liner is removed prior to use.
(b) Coating Process by Electrostatic Powder Deposition Onto a Substrate: Feasibility of coating the galvanic particulates onto a substrate with the electrostatic powder deposition technique was demonstrated using a commercial high voltage powder electrostatic coating system (HV Powder Coating System, purchased from Caswell, Inc., Lyons, New Yortk). The galvanic particulate and hydrocolloid materials, and sample preparation procedure were same as Example 2a. The voltage setting of the HV Powder Coating System was set at 45 kV, and compressed air was controlled at 15 psi (pounds-per-inch). The simple and high speed coating process resulted in a uniform coating of the galvanic powder on the hydrocolloid sheet was uniform.

### Example 3- In vitro Efficacy of Galvanic Particulates Against MRSA, Yeast, and Bacteria

Galvanic particulates containing-agar discs were made by suspending the galvanic particulates from Example 1(a) in 2 ml of 47°C sterile distilled water mixed with 8 ml of melted agar. The mixture was then poured into a 100 x 15 mm petri dish. The mixture solidified in the petri dish, and the galvanic particulates were immobilized and evenly distributed in the agar. Smaller agar discs were cut out from the galvanic particulate-containing agar with a sterile cork borer (inner D=12.2.mm), and used for further testing of the galvanic particulates.

The agar discs (D = 12.2 mm, thickness = 1.2 mm), containing the galvanic particulates at a concentration of either 0.5% or 1%, were placed on an agar plate surface inoculated with about 6 log CFU of indicator microorganisms. The plates were incubated at 37°C for 24 hours. The zone of inhibition (distance in mm from edge of disc and edge of clear no growth zone) was measured with a digital caliper. Duplicate samples were used for this test. The results are depicted in Table 1.

**Table 1**

| **Strains** | **Class** | **Zone of inhibition (mm) 0.5%** | **Zone of inhibition (mm) 1%** |
|---|---|---|---|
| MRSA (Methicillin Resistant Staphylococcus aureus 33593) | Gram+ Bacteria | 1.3 | 2.9 |
| MRSE (Methicillin Resistant Staphylococcus epidermidis 51625) | Gram+ Bacteria | 1.8 | 3.6 |
| Candida albicans 10231 | Yeast | 0.9 | 2.0 |
| Pseudomonas aeruginosa 9027 | Gram- Bacteria | 0.4 | 1.2 |
| *Corynebacterium aquaticum* 14665 | Gram+ Bacteria | 1.0 | 1.4 |
| Corynebacterium jeikeium 43734 | Gram+ Bacteria | 1.9 | 3.3 |
| Staphylococcus haemolyticus 29970 | Gram+ Bacteria | 1.0 | 1.3 |
| *Micrococcus lylae* 27566 | Gram+ Bacteria | 1.0 | 2.3 |

| | | | |
|---|---|---|---|
| * Results are means of duplicate samples | | | |

These results indicated that galvanic particulates were inhibitory against a wide-range of microorganisms, including antibiotic resistant bacteria (MRSA and MRSE), yeast (*Candida albicans*), and odor-producing species (*Corynebacterium aquaticum, C. jeikeium, Staphylococcus haemolyticus, Micrococcus lylae, S. epidermidis*). *This in vitro* efficacy shows the promises of using galvanic particulates for wound infection products, vaginal health products, and odor-reducing products.

### Example 4- Efficacy of Galvanic Particulates Against MRSA and C. albicans Versus Metal Salt Controls

Agar discs containing copper-zinc galvanic particulates from Example 1(a) or zinc acetate at a concentration of 0.1%, 0.5%, or 1% were exposed to about 6 log CFU of MRSA or C. *albicans* in saline in microwell plate and incubated at 37°C and 200 rpm for 24 hrs. Plate count was performed to enumerate the viable microorganisms after the incubation. Log reduction was defined as the log difference of the inoculum before and after the incubation with the test articles (e.g., a log reduction of 6 for the inoculum of 6 log means all the inoculum were killed, and a log reduction of 3 for the inoculum of 6 log means 50% of the inoculum were killed). The results are set forth below in Table 2.

**Table 2**

| | **LOG REDUCTION** | | | |
|---|---|---|---|---|
| | **C. albicans** | | **MRSA** | |
| **Concentration of test material** | **Galvanic particulates** | **Zinc Acetate** | **Galvanic particulates** | **Zinc Acetate** |
| **0.10%** | 6.5 | 2.2 | 2.4 | 1.7 |
| **0.50%** | 6.5 | 2.9 | 6.7 | 3.2 |
| **1.00%** | 6.5 | 4.7 | 6.7 | 5.1 |

Results show that the galvanic particulates have significantly more antimicrobial potency that zinc acetate, a metal salt control.

### Example 5- Comparison of Antimicrobial Activity Against MRSA and VRE of Galvanic Particulates Versus Copper Metal and Zinc Metal Powders

Agar discs with either galvanic particulates from Example 1(a) copper metal powders, zinc metal powders, or a control TSA only agar disc were inoculated with either 10e3 VRE or 10e5 MRSA. The zone of inhibition was evaluated. Results, reported in Table 3, indicated that 1% copper-zinc galvanic particulates inhibited growth of the inoclum completely, while the control, copper metal powder, and zinc metal powder discs showed no inhibition.

**Table 3**

| **Test material** | **MRSA (10e3 inoculum)** | **MRSA (10e5 inoculum)** |
|---|---|---|
| Control: TSA agar disc only | No inhibition | No inhibition |
| 1% w/w Copper metal | No inhibition | No inhibition |
| 1% w/w Zinc metal | No inhibition | No inhibition |
| 1% w/w Copper-zinc galvanic particulates | Inhibition | Inhibition |

### Example 6- Comparison of Antimicrobial Activity Against C. albicans and MRSA of Galvanic Particulates Versus Copper Acetate and Zinc Acetate.

Zone of inhibition testing was performed on agar discs containing copper-zinc galvanic particulates from Example 1(a) at 0.5%, Zn acetate at 0.5%, and Cu acetate at 0.1%. The discs were placed on TSA agar surface, inoculated with about 6 log CFU of MRSA or C. *albicans,* and incubated at 37°C for 24 hr. It was found that with both MRSA and C. albicans, the 0.5% galvanic particulates showed a significant, visible zone of inhibition. The 0.5% zinc acetate showed a smaller zone of inhibition, approximately one half the radius of the zone produced with the 0.5% galvanic particulates. The 0.1% copper acetate did not show any visible zone of inhibition with MRSA nor C. albicans.

### Example 7- Comparison of Galvanic Particulates and Zinc Acetate and Copper Acetate by Agar Disc Microwell Assay

Agar discs containing 0.1% copper coated zinc galvanic particulates from Example 1(a) or zinc acetate at 1% or copper acetate at 0.1% were exposed to about 6 log CFU of MRSA or C. *albicans* in saline in microwell plates, and incubated at 37°C, 200 rpm for 24 hr. Plate count was performed to enumerate the viable microorganisms after the incubation. Log reduction was defined as the log difference of the inoculum before and after the incubation with the test articles. The results are depicted below in Table 4.

**Table 4**

| | **LOG REDUCTION** | |
|---|---|---|
| | **C. albicans** | **MRSA** |
| 1% Galvanic Particulates | 6.4 | 6.7 |
| 1% Zinc Acetate | 4.7 | 5.1 |
| 0.1% Copper Acetate | 0.3 | 0.2 |

### Example 8 - Evaluation of the Long-lasting, Sustained Efficacy of Galvinc Particulates Compared to Zinc Acetate

Agars discs containing either galvanic particulates as described in Example 1(a) or zinc acetate at 1% were placed on TSA agar surface inoculated with about 6 log CFU of MRSA or C. *albicans* and incubated at 37°C for 24 hr (day-1). After the incubation the agar discs were observed for zone of inhibition, then removed from the plates and placed onto a newly inoculated TSA plates with the same inoculum and incubated for 24 hr (day-2). It was found that on day 1, both the galvanic particulate disc and zinc acetate disc produce a zone of inhibition against C. albicans and MRSA, and the zone produced by the galvanic particulates was larger than that of the zinc acetate disc. However, on day 2 only the disc containing the galvanic particulates demonstrated a visible zone of inhibition; the disc containing the zinc acetate did not show any inhibition. This demonstrates that the galvanic particulates have antimicrobial or inhibitory effects over sustained periods of time.

### Example 9- Immunomodulation of Human T-Cell Cytokine Release Stimulated with PHA

The ability of the galvanic particulates from Example 1(a) to modulate immune responses was illustrated by their ability to reduce the production of cytokines by activated human T-cells stimulated with the T-cell receptor (TCR) activating agent phytohaemagglutinin (PHA) in the following assay.

Human T-cells were collected from a healthy adult male via leukopheresis. The T-cells were isolated from peripheral blood via Ficol gradient, and the cells were adjusted to a density of 1x10⁶ cells/mL in serum free lymphocyte growth medium (ExVivo-15, Biowhittaker, Walkersville, MD). Human T-cells were stimulated with 10µg/mL PHA in the presence or absence of test compounds following published method (Hamamoto Y., et al. Exp Dermatol 2:231-235, 1993). Following a 48-hour incubation at 37°C with 5% CO₂, supernatant was removed and evaluated for cytokine content using commercially available multiplex cytokine detection kit. The results are depicted in Table 7.

**Table 7**

| | **Cytokine Release** | |
|---|---|---|
| **Treatment** | **IL-2 (pmol/ml)** | **Percent (%) Reduction** |
| Unstimulated | 2.8 ± 4.0 | - |
| PHA Stimulated | 563.2 ± 60.0 | - |
| PHA + Copper Metal (100 ug/ml) | 498.9 ± 64.4 | 11.4% |
| PHA + Zinc Metal (100 ug/ml) | 456.8 ± 11.1 | 18.9% |
| PHA +Zinc Chloride (100 ug/ml) | 566.3 ± 20.6 | -0.6% |
| PHA +Copper (II) Acetate (100 ug/ml) | 312.9 ± 96.8 | 44.4% |
| PHA + Galvanic particulates (100 ug/ml) | 10.15 ± 3.5 | 98.2% |
| Hydrocortisone (Pos. Control 100 ug/ml) | 7.69 ± 5.64 | 98.6% |

(where IL-2 = Interleukin-2 (Cytokine)).

The galvanic particulates were found to be able to modulate the release of inflammatory mediators induced by T-cell stimulation. Furthermore, the anti-inflammatory activity was greater than that of copper metal powder, zinc metal powder, copper ion (Copper (II) Acetate), or zinc ions (Zinc Chloride) alone.

### Example 10- Inhibition of NF-kB Activation

Nuclear Factor Kappa Beta (NF-kB) is a transcription factor that binds to the NF-kB binding site on the promoter region of pro-inflammatory genes, such as COX-2 and Nitric Oxide Synthase (iNOS) (Bell S, et al (2003) Cell Signal.;15(1):1-7). NF-kB is involved in regulating many aspects of cellular activity, in stress, injury and especially in pathways of the immune response by stimulating synthesis of pro-inflammatory proteins, such as Cycloxygenase-2 (COX-2), thus leading to inflammation (Chun KS, t al. (2004) Carcinogenesis 25:445-454.; Fenton MJ (1992) Int J Immunopharmacol 14:401-411). NF-kB itself is induced by stimuli such as pro-inflammatory cytokines (e.g. TNF-alpha and IL-1beta), bacterial toxins (e.g. LPS and exotoxin B), a number of viruses/viral products (e.g. HIV-1, HTLV-I, HBV, EBV, and Herpes simplex), as well as pro-apoptotic and necrotic stimuli (e.g., oxygen free radicals, UV light, and gamma-irradiation). Inhibition of NF-kB activation is likely to reduce inflammation by blocking the subsequent signaling that results in transcription of new pro-inflammatory genes.

Solar ultraviolet irradiation activates the transcription factor NF-kB, inducing the production of matrix metalloproteinases that can lead to degradation of matrix proteins such as elastin and collagen. Inhibitors of NF-kB are likely to inhibit the subsequent signaling that results in the presence of MMPs in the dermal matrix, and the more of the pathway that is inhibited, the more likely there will be no induction of MMPs. Recently inhibition of the NF-kB pathway has shown to result in a subsequent induction in collagen synthesis (Schreiber J, et al. (2005) Surgery. 138:940-946). Thus, inhibition of NF-kB activation may also provide anti-aging benefits to skin by increasing collagen synthesis.

To evaluate the activity of galvanic particulates from Example 1(a) in blocking NF-kB activation, FB293 cells, a stable transfected human epithelial cell line containing the gene reporter for NF-kB was obtained from Panomics (Fremont, CA), were used. FB293 cells were plated at a density of 5x10⁴ cells/mL in Dulbecco's modified Eagle's medium (DMEM) supplemented with 10% fetal bovine serum (Invitrogen, San Diego, CA). FB293 cells were stimulated with 50ng/mL 12-O-tetradecanoylphorbol 13-acetate (TPA)(Sigma St Louis, MO) in the presence or absence of galvanic particulates. Following a 24 hour incubation at 37°C with 5% CO₂, cells were lysed with 40 µl of reporter lysis buffer (Promega, Madison, WI). A 20-µl aliquot of the lysate was assayed using a luciferase assay kit (Promega) and counted for 10 seconds in a Lmax luminometer (Molecular Devices, Sunnyvale, CA) with the data represented as the relative light unit/second. Galvanic particulates were found to inhibit NF-kB activation as shown in Table 8.

**Table 8**

| | **NF-kB Gene Reporter Activation** **(Luminescence)** | **Percent Inhibition** |
|---|---|---|
| Untreated | 4.06 ± 0.6 | - |
| TPA (10ng/ml Stimulated | 28.46 ± 2.21 | - |
| TPA + Galvanic particulates (100 ug/ml) | 3.20 ± 1.98 | 88.7% |
| UV (10 kJ) Stimulated | 11.45 ± 1.89 | - |
| UV (10 kJ) + Galvanic particulates (100 ug/ml) | 5.51 ± 1.74 | 51.6% |

Galvanic particulates, thus, were found to substantially reduce NF-kB activation. This example demonstrates that galvanic particulates can modulate the production of inflammatory mediators, which contribute to inflammation of the skin. This example also demonstrates that galvanic particulates may also protect elastin and collagen fibers from damage and degradation that can lead to aging of the skin.

### Example 11 - Anti-Inflammatory Activity on Release of UV-Induced Pro-inflammatory Mediators on Reconstituted Epidermis

The effect of galvanic particulates was evaluated for topical anti-inflammatory activity on human epidermal equivalents. Epidermal equivalents (EPI 200 HCF), multilayer and differentiated epidermis consisting of normal human epidermal keratinocytes, were purchased from MatTek (Ashland, MA). These epidermal equivalents were incubated for 24 hours at 37°C in maintenance medium without hydrocortisone. Equivalents were topically treated (2mg/cm²) with galvanic particulates (1 mg/ml) from Example 1(a) in 70% ethanol/30% propylene glycol vehicle 2 hours before exposure to solar ultraviolet light (1000W-Oriel solar simulator equipped with a 1-mm Schott WG 320 filter; UV dose applied: 70 kJ/m² as measured at 360nm). Equivalents were incubated for 24 hours at 37°C with maintenance medium then supernatants were analyzed for IL-8 cytokine release using commercially available kits (Upstate Biotechnology, Charlottesville, VA). The results are depicted in Table 9.

**Table 9**

| **Treatment (Dose, as % w/v)** | **Mean +/- Std Dev of IL-1A Release (ng/ml)** | **Percent Inhibition of Skin Inflammation** |
|---|---|---|
| Untreated, No UV | 223.5 ± 168.0 | - |
| UV (60 KJ), Vehicle Treated | 944.9 ± 205.3 | - |
| UV (60 KJ) + Galvanic particulates (1 mg/ml) | 477.7 ± 177.9** | 50.4% |

| | | |
|---|---|---|
| ** Indicates significant difference from UV, Vehicle treated using a student's t-Test with significance set at P<0.05. | | |

Based on this example, topical application of galvanic particulates was able to significantly reduce the UV-stimulated release of inflammatory mediators. Therefore, galvanic particulates would be expected to provide an effective the anti-inflammatory benefit when applied to skin.

### Example 12 - Reduction of Methyl Nicotinate-Induced Skin Erythema

Methyl nicotinate (methyl 3-pyridinecarboxylate) is a known vasodilator causing an increased cutaneous blood flow upon its application on the skin. See, Guy R. H., Arch. Dermatol Res (1982) 273:91-95. In this experiment, between 10 mM-solution of methyl nicotinate (Aldrich Chemical, St. Louis, Mo.) was topically applied for 30 seconds under occlusion (2.5 cm disk, Hill Top Research Inc, Cincinnati, Ohio) on the volar forearm of volunteers based on the method of Jumbelic et al. (Skin Pharmacol Physiol. (2006) 19:147-152). Galvanic particulates (10 mg/ml) from Example 1(a) in 70% ethanol/30% propylene glycol vehicle were topically applied after induction of erythema by methyl nicotinate challenge. Redness was assessed by diffuse reflectance spectroscopy. See Kollias N, et al., Photochem Photobiol. (1992) (56):223-227. An Ocean Optics diode array spectrophotometer (Dunedin, Fla.) connected to a HP laptop computer through a USB port was used to control the experiment and to collect and analyze the spectral data.

An optic fiber bundle was used to conduct the light from the lamp to the skin and transmit the reflectance measurements back from the skin to the spectrophotometer. The results are depicted in Table 10.

**Table 10**

| **Treatment (Dose, as % w/v)** | **Mean +/- Std Dev of Apparent Hemoglobin** | **Percent Inhibition of Skin Erythema** |
|---|---|---|
| Placebo | 0.72 ± 0.22 | - |
| Galvanic particulates (10mg/ml) | 0.43 ± 0.19 ** | 40.2% |

| | | |
|---|---|---|
| ** Indicates significant difference from Placebo treated using a student's t-Test with significance set at P<0.05. | | |

These results indicate that topical application of galvanic particlulates reduced the erythema on a methyl nicotinate-induced human redness model.

### Example 13- Stimulation of Hydrogen Peroxide Production by Galvanic Particulates

Hydrogen peroxide (H₂O₂) has strong oxidizing properties and is therefore a powerful bleaching agent. Hydrogen peroxide is also an effective anti-bacterial, anti-fungal, and antiviral compound that is even effective against methicillin resistant Staphylococcus aureus (MRSA) isolates (Flournoy DJ, Robinson MC. (1990) Methods Find Exp Clin Pharmacol. 12:541-544). In addition, rinsing the oral cavity with a solution of hydrogen peroxide results in a significant reduction of aerobic and anaerobic bacteria in saliva (Matula C, Hildebrandt M, Nahler G. (1988) J Int Med Res.;16:98-106). The reduction in bacteria in the oral cavity can help reduce the incidence of gingivitis.

Peroxides have been used in tooth whitening for more than 100 years, and hydrogen peroxide is one of the most commonly used active agents used in tooth whitening. (Li Y. (1996) Food Chem Toxicol. 34:887-904). Hydrogen peroxide is also an effective vasoconstrictor that can reduce the appearance of dark circles, and result in a skin whitening effect. (Stamatas GN, Kollias N. (2004). J Biomed Opt. 9:315-322; Goette DK, Odom RB. (1977) South Med J. 70:620-622.).

The ability of galvanic particulates from Example 1(a) to induce the production of hydrogen peroxide was illustrated in the following assay. Human keratinocyte cells were seeded in assay plates at identical densities and incubated for 48 hours at 37°C with 5% CO₂. To detect hydrogen peroxide production, keratinocytes were loaded for a 30-minute incubation period with 5µM of the hydrogen peroxide-sensitive fluorescent probe 5-(and-6)-chloromethyl-2',7'-dichlorodihydrofluorescein diacetate, acetyl ester (CM-H2DCFDA, Invitrogen Carlsbad, CA). Cells were treated with galvanic particulates or zinc or copper metal powders alone over increasing amounts of time. Treatment of control wells with 0.03% hydrogen peroxide served as a positive control. Hydrogen peroxide production was quantitated using a fluorescent plate reader set at wavelengths 485 excitation/530 emission. The results are depicted in Tables 11 and 12.

**Table 11**

| **Compound** | **Baseline** | **30 Minutes** | **60 Minutes** | **200 Minutes** | **240 Minutes** |
|---|---|---|---|---|---|
| Untreated | 42.3 ± 9.3 | 61.4 ± 13.9 | 88.1 ± 29.5 | 215.4 ± 125.8 | 243.9 ± 138.9 |
| Galvanic particulates (1%) | 77.3 ± 16.2 | 385.5 ± 98.6** | 726.6 ± 158.6** | 877.6 ± 186.3** | 842.2 ± 176.002** |
| H₂O₂ (0.03%) | 98.1 ± 4.4 | 416.6 ± 61.3** | 591.4 ± 82.7** | 1117.5 ± 153.8** | 1214.8± 149.7** |

| | | | | | |
|---|---|---|---|---|---|
| ** Indicates significant difference from baseline hydrogen peroxide levels at that timepoint using a student's t-Test with significance set at P<0.05. | | | | | |

**Table 12**

| **Compound** | **60 Minutes** |
|---|---|
| Copper Metal (0.1%) | 62.7 ± 4.27 |
| Zinc Metal (0.1%) | 76.4 ± 10.31 |
| Galvanic particulates (0.1 %) | 190.5± 0.84 |

Based on this example, galvanic particulates were able to significantly induce the production of hydrogen peroxide. Furthermore, the production of hydrogen peroxide generated by galvanic particulates was substantially greater than that of copper metal powders or zinc metal powders alone. Therefore, galvanic particulates would be expected to provide an effective skin lightening, tooth whitening, and anti-bacterial activity when applied to skin.

### Example 14 - Example of Topical Formulations

(a) Topical Gel: A topical gel formulation of Table 13 containing galvanic particulates of Example 1 can be manufactured as follows:

**Table 13**

| **INCI Name** | **% (w/w) in Formulation** |
|---|---|
| Propylene Glycol | 0-60 |
| Hydroxyethyl Acrytate/Sodium Acryloyldimethyl Taurate Copolymer | 0-5 |
| Glycerin 99.7% | 0-50 |
| PEG-12 Dimethicone | 0-50 |
| Cyclopentasiloxane | 0-50 |
| Galvanic Particluates | 0.01-5 |

Into a main vessel, the Propylene Glycol and Glycerin were added. The Hydroxyethyl Acrylate/Sodium Acryloyldimethyl Taurate Copolymer was then added and mixed until uniform, following which it was heated to 40C until the composition clears and no particles are present. The batch is then cooled to 40C, following which the remaining ingredients were added and mixed until uniform and further cooled.
(b) Topical Stick: A topical gel formulation of Table 14(a) containing galvanic particulates of Example 1 can be manufactured as follows:

**Table 14(a)**

| **INCI NAME** | **% (w/w) in Formulation** |
|---|---|
| cyclomethicone | 0-75 |
| propylene glycol | 0-50 |
| sodium stearate | 0-50 |
| PEG 400 | 0-100 |
| Ozokerite | 0-30 |
| Paraffin | 0-50 |
| Cetyl Alcohol | 0-50 |
| Galvanic Particluates | 0.01-5 |

In main vessel, all ingredients except for propylene glycol and galvanic particulates were combined and heated to 85-90°C until completely melted. In a separate container, the propylene glycol and galvanic particulates were mixed until the particulates were evenly dispersed. Once the composition in the main vessel was uniform, the propylene glycol & galvanic particulate mixture was into the main batch at 85C. The entire batch was mixed until uniform and then cooled to 65- 70C.
(c) Dual chamber or dual phase topical product: A topical composition in a dual-chamber package for the purpose of dispensing 2 separate formulations that may otherwise be unstable if stored in a single chamber over time can be made. A dual-chamber topical composition with one anhydrous composition in a single chamber, separated from an aqueous or conducting composition in a second chamber can be made as follows. Chamber 1 contains the composition described in Example 14(a). Chamber 2 contains the following formulation of Table 14(b).

**Table 14(b)**

| **INCI NAME** | **% (w/w) in Formulation** |
|---|---|
| Water | 0-99 |
| Acrylates / C10-30 Alkyl Acrylate Cross-Polymer) | 0.05-2 |
| Benzalkonium Chloride | 0-0.1 |
| Tetrahydroxypropyl Ethylenediamine | 0-5 |

The formulations are loaded into a dual-chamber package, with each formulation in a separate chamber. At the point of use, the formulations are dispensed and mixed onto the site of application. An alternate way of dispensing the formulations is in a two-step process, whereby the first formulation is dispensed onto the skin followed by the second formulation. The two are mixed together and applied on the desired application site.

### Example 15 - Anti-fungal Effect

The galvanic particulates of Example 1(a) were evaluated in an in vitro onychomycosis model similar to that described in Yang, et al. Mycopathologia 148: 79-82, 1999. In order to simulate the foot onychomychosis, cow hoofs were used. Hoofs were punched into plates of 1.3 cm in diameter and then sterilized in an autoclave. The hoof plates were placed in sterile Petri dishes with their external face on sterile filter paper soaked with one of the antifungal preparations or with sterile water as controls. An agar block from a dermatophyte culture was implanted on the internal face. The whole apparatus was placed in a larger Petri dish containing sterile water to prevent dehydratation. After inoculation, the dermatophytes were moistened with 5 microliters of Sabouraud broth on a daily basis. The broth was deposited with a micro-pipette on the internal face of the hoof plate at the base of the agar block. The experimental material was placed on the hoof system at day 0, and the fungal growth was monitored daily, to determine the first day that the fungus grew through the nail. The date of appearance and amount of growth breakthrough was recorded. Hydrocolloid coated with 3.6 mg/cm² galvanic particulates was compared to untreated control. All samples were replicated 3 times.

The results showed that the first breakthrough of fungal growth with the untreated control was 2 days, while the first breakthrough with the galvanic particulates was 5 days. This indicates that the galvanic particulates inhibit fungal growth or have anti-fungal activity.

### Example 16 - Anti-Aging Benefits of Galvanic Particulates

Aging of the skin is a complex phenomenon resulting from the interaction of several intrinsic and extrinsic factors. Intrinsic aging is an inevitable, genetically programmed process. Among extrinsic influences (e.g., wind, heat, cigarette smoke, chemicals, etc.), ultraviolet radiation appears to be the single most important factor associated with aging of the skin. As skin ages, it generally loses elasticity as it ages. This is attributed to skin thinning and loss of elastin and collagen in the dermal matrix, as well as losses in the subcutaneous tissue (such as fat layers and muscle mass), which are expressed as sagging of the skin. The mechanical properties of the skin are, in particular, heavily influenced by the microstructural arrangement of collagen and elastin in the dermal matrix. Elastin is a critical component of extracellular matrix, and is especially abundant in tissues subject to physical deformations, such as skin. Galvanic particulates were found to effectively inhibit the enzymes that degrade elastin in the skin and thus would be expected to enhance the elasticity of the skin.

Human leukocyte elastase (HLE) was purchased from Sigma (St. Louis, Mo.), and reconstituted at 1 unit/ml in phosphate buffered saline (PBS, Invitrogen life Technologies, Carlsbad, Calif.). Soluble bovine neck ligament elastin labeled with BODIPY FL dye was purchased from Molecular Probes, Inc. (Eugene, Oreg.), such that the fluorescence was quenched in the conjugate, and could be activated upon elastase digestion. Human leukocyte elastase (0.0625 U/ml), elastin substrate (25 µg/ml), and increasing concentrations of test material were incubated for two hours at 37C. Fluorescence was measured at excitation at 490 nm and emission at 520 nm using a fluorescent plate reader Gemini from Molecular Devices (Sunnyvale, Calif.). Background fluorescence of substrate alone had been subtracted from each measurement.

Galvanic particulates of Example 1(a) inhibited HLE activity in a dose dependent manner as shown in Table 15. As low as 10 ug/ml of 'Galvanic particulates' resulted in approximately 50% reduction in HLE activity. This example demonstrates that 'Galvanic particulates' can protect elastin fibers from damage and degradation.

**Table 15**

| **Galvanic particulates (ug/ml)** | **Elastase Inhibition (%)** |
|---|---|
| 0 | 0 |
| 1.0 | 46.5 |
| 10 | 48.7 |
| 100 | 53.8 |
| 1000 | 60.8 |

### Example 17: Galvanic particulates reduces Pigmentation in Pigmented Epidermal Equivalents

Regulation of pigmentation is an important aspect of improving skin eveness, skin appearance, and skin tone. Galvanic particulates of Example 1(a) was also tested for its ability to reduce pigmentation in pigmented epidermal equivalents. The pigmented epidermal equivalents contain human normal melanocytes, together with normal, human-derived epidermal keratinocytes, which have been cultured to form a multi-layered, highly differentiated model of the human epidermis. The epidermal equivalents used were EpiDerm™ reconstructed human epidermis from MatTek Corp. (Ashland, MA). Pigmented epidermal equivalents (MEL-A, consists of normal human keratinocytes pooled from variety of phototype skins and normal human melanocytes derived from Asian donor) were treated with galvanic particulates at 1% suspended in water for six days and samples were harvested on the seventh day of the study. The harvested equivalents were stained with Fontana-Mason (F&M) (Sheenan DC, Hrapckak BB, eds: Theory and practice of Histo-Thchnology (St Louis: CV Mosby, 1980) pp 223-277). F&M staining identifies silver nitrate reducing activity, which, in skin, identifies melanin.

The galvanic particulates was suspended in water at 1% (w/v) and applied topically once daily for 6 days. On the seventh day of the study, the equivalents were fixed, sectioned and F&M stained. F&M-stained histological sections were evaluated for the change in pigment deposition. All images were obtained and analyzed with Image Pro Plus 4.0 software (Media Cybernetics, Silver Spring, MD). Parameters measured were surface area of stained material within melanocyte and keratinocytes and the total surface area of the cells in the culture, and the relative pigmented area was calculated. A value of 100% was assigned to untreated controls, and values of treatment groups were normalized to their relevant controls. Data are presented with standard deviation (SigmaPlot^{®} 5.0, SPSS Science, Chicago, IL). At least three sections per equivalent, three equivalents per experiment were processed. Each experiment was repeated three times.

Table 16 shows the results of representative data, normalized for their relative controls (H₂O), demonstrating that galvanic particulates treatment reduced pigmentation. This table demonstrates the specificity of the compositions of this invention in reducing pigmentation (e.g., reducing pigmentation by up to 51 %).

**Table 16**

| **Test Material** | **Concentration** | **% Melanin** |
|---|---|---|
| Control (H₂O) | - | 100% |
| Galvanic Particulates | 1 % (W/V) | 51+/- 5% |

### Example 18 - In-vitro Depigmentation

Regulation of pigmentation is an important aspect of improving skin eveness, skin appearance, and skin tone. The galvanic particulates as described in Example 1(a) was tested in an in vitro pigmentation model with pigmented epidermal equivalents. Pigmented epidermal equivalents contain human normal melanocytes, together with normal, human-derived epidermal keratinocytes, which have been cultured to form a multi-layered, highly differentiated model of the human epidermis. The 0.01% galvanic particulates was suspended in water and placed onto the epidermal equivalents (4.2 cm²). The study included a placebo control of water alone. The epidermal equivalents were monitored for 7 days. Histology results show that the galvanic particulates treatment reduced melanin deposition in skin equivalents by Day 7 compared to the placebo. This indicates that the galvanic particulates can have skin depigmentation benefits.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

### Example 19 - Stimulation of Hydrogen Peroxide Production by Galvanic Particulates

Hydrogen peroxide (H₂O₂) has strong oxidizing properties and is therefore a powerful bleaching agent. Hydrogen peroxide is also an effective anti-bacterial, anti-fungal, and anti-viral compound that is even effective against methicillin resistant Staphylococcus aureus (MRSA) isolates (Flournoy DJ, Robinson MC. (1990) Methods Find Exp Clin Pharmacol. 12:541-544). In addition, rinsing the oral cavity with a solution of hydrogen peroxide results in a significant reduction of aerobic and anaerobic bacteria in saliva (Matula C, Hildebrandt M, Nahler G. (1988) J Int Med Res.;16:98-106). The reduction in bacteria in the oral cavity can help reduce the incidence of gingivitis.

Peroxides have been used in tooth whitening for more than 100 years, and hydrogen peroxide is one of the most commonly used active agents used in tooth whitening. (Li Y. (1996) Food Chem Toxicol. 34:887-904). Hydrogen peroxide is also an effective vasoconstrictor that can reduce the appearance of dark circles, and result in a skin whitening effect. (Stamatas GN, Kollias N. (2004). J Biomed Opt. 9:315-322; Goette DK, Odom RB. (1977) South Med J. 70:620-622.).

The ability of galvanic particulates from Example 1(b) to induce the production of hydrogen peroxide was illustrated in the following assay. Human keratinocyte cells were seeded in assay plates at identical densities and incubated for 48 hours at 37°C with 5% CO₂. To detect hydrogen peroxide production, keratinocytes were loaded for a 30-minute incubation period with 5µM of the hydrogen peroxide-sensitive fluorescent probe 5-(and-6)-chloromethyl-2',7'-dichlorodihydrofluorescein diacetate, acetyl ester (CM-H2DCFDA, Invitrogen Carlsbad, CA). Cells were treated with galvanic particulates or zinc or copper metal powders alone over increasing amounts of time. Treatment of control wells with 0.03% hydrogen peroxide served as a positive control. Hydrogen peroxide production was quantitated using a fluorescent plate reader set at wavelengths 485 excitation/530 emission. The results are depicted in Tables 17 and 18.

**Table 17**

| **Compound** | **Baseline** | **30 Minutes** | **60 Minutes** | **200 Minutes** | **240 Minutes** |
|---|---|---|---|---|---|
| Untreated | 42.3 ± 9.3 | 61.4 ± 13.9 | 88.1 ± 29.5 | 215.4 ± 125.8 | 243.9 ± 138.9 |
| Galvanic particulates (1%) Ethanol Process (Example 1b) | 77.3 ± 16.2 | 385.5 ± 98.6** | 726.6 ± 158.6**‡ | 877.6 ± 186.3**‡ | 842.2 ± 176.2**‡ |
| Galvanic | 65.4 ± | 288.1 ± | 473.2 ± | 634.7 ± | 636.1 ± 64.2** |
| particulates (1%) Water Process (Example 1(a)) | 10.1 | 28.2** | 41.4** | 57.6** | |
| H₂O₂ (0.03%) | 98.1 ± 4.4 | 416.6 ± 61.3** | 591.4 ± 82.7** | 1117.5 ± 153.8** | 1214.8± 149.7** |

| | | | | | |
|---|---|---|---|---|---|
| ** Indicates significant difference from baseline hydrogen peroxide levels at that timepoint using a student's t-Test with significance set at P<0.05. ‡ : Indicates significant difference from Water Process produced Galvanic particulates hydrogen peroxide levels at that timepoint using a student's t-Test with significance set at P<0.05. | | | | | |

**Table 18**

| **Compound** | **60 Minutes** |
|---|---|
| Copper Metal (0.1%) | 62.7 ± 4.27 |
| Zinc Metal (0.1%) | 76.4 ± 10.31 |
| Galvanic particulates (0.1%) | 190.5± 0.84 |

Based on this example, galvanic particulates were able to significantly induce the production of hydrogen peroxide. The production of hydrogen peroxide generated by galvanic particulates was substantially greater than that of copper metal powders or zinc metal powders alone. Furthermore, the production of hydrogen peroxide generated by galvanic particulates created using the Ethanol process was substantially greater than that of galvanic particulates created using the water process. Therefore, galvanic particulates created using the Ethanol process would be expected to provide an effective skin lightening, tooth whitening, and anti-bacterial activity when applied to skin.

### Example 20 - Controlling rate of reaction, quality, and activity of galvanic particulates

Changing the conditions of the metal plating of one metal onto another has been shown in Example 19 to affect the activity of galvanic particulates. The polarity of the reaction medium and presence of other agents such as complexing and chelating agents, therefore, can be adjusted to create galvanic particulates of varying properties, including but not limited to coating thickness, coating density, coating pattern, and/or rate of reaction. The ability to control the rate of plating copper onto zinc powders is illustrated with the following example. The process described in Example 1(b) was performed with various types of 0.61 % w/w copper acetate solutions outlined in Table 19. In Table 19, the reaction time refers to the time it took for the copper to completely deposit onto the zinc powder, indicated by the copper salt solution changing from blue to clear.

**Table 19**

| **% water** | **% ethanol** | **reaction time (hr)** |
|---|---|---|
| 0 | 100 | 48.00 |
| 10 | 90 | 5.67 |
| 15 | 85 | 0.50 |
| 17 | 83 | 0.52 |
| 18 | 82 | 0.50 |
| 20 | 80 | 0.00 |

Based on this example, the rate of the coating reaction can be regulated by the polarity of the metal salt solution. Example 19 shows that the activity of the resulting galvanic particulates is affected by manufacturing conditions.

Numbered embodiments of the invention:
1. A galvanic particulate comprising a first conductive material and a second conductive material, wherein both said first conductive material and said second conductive material are exposed on the surface of said particulate, wherein the particle size of said particulate is from about 10 nanometers to about 100 micrometers, wherein the second conductive material comprises from about 0.01 percent to about 10 percent, by weight, of the total weight of said particulate, and wherein the difference of the standard potentials of the first conductive material and the second conductive material is at least about 0.2 V.
2. A galvanic particulate of embodiment 1, wherein said particulate comprises said first conductive material and wherein the surface of said particulate is partially coated with said second conductive material.
3. A galvanic particulate of embodiment 1, wherein said particulate comprises at least 95 percent, by weight, of said first conductive material and said second conductive material.
4. A galvanic particulate of embodiment 1, wherein said first conductive material is zinc.
5. A galvanic particulate of embodiment 1, wherein said second conductive material is copper or silver.
6. A galvanic particulate of embodiment 4, wherein said second conductive material is copper or silver.
7. A galvanic particulate of embodiment 2, wherein said particulate is partially coated with a third conductive material.
8. A galvanic particulate of embodiment 6, wherein said particulate comprises at least 95 percent, by weight, of said first conductive material, said second conductive material, and said third conductive material.
9. A galvanic particulate of embodiment 6, wherein said first conductive material is zinc, said second conductive material is copper and said third conductive material is silver.
10. A galvanic particulate of embodiment 8, wherein said second conductive material is copper and said third conductive material is silver.
11. A method of manufacturing a particulate of embodiment 2, wherein said method comprises contacting a particulate of said first conductive material with a solution comprising a salt of the said second conductive material.
12. A method of embodiment 11, wherein said method comprises flowing said solution over said particulate.
13. A method of embodiment 11, wherein said solution comprises an organic solvent.
14. A method of embodiment 13, wherein said organic solvent is selected from the group consisting of an alcohol, a glycol, or glycerin.
15. A composition comprising a particulate of embodiment 1 and a bio-absorbable polymer.
16. A composition of embodiment 15, wherein said bio-absorbable polymer is selected from the group consisting of collagen, hyaluronic acid, or a mixture thereof.
17. An oral dosage form comprising a particulate of embodiment 1 and a pharmaceutically acceptable carrier.
18. A method of treating a gastrointestinal disorder, said method comprising orally administering the oral dosage form of embodiment 17.
19. A method of embodiment 18, wherein said gastrointestinal disorder is selected from the group consisting of ulcers, diarrhea, and gastrointestinal pain.
20. A method of killing drug resistant microorganism, said method comprising contacting said microorganism with a composition comprising a galvanic particulate comprising a first conductive material and a second conductive material, wherein both said first conductive material and said second conductive material are exposed on the surface of said particulate, and wherein the difference of the standard potentials of the first conductive material and the second conductive material is at least about 0.2 V.
21. A method of embodiment 20, wherein the particle size of said particulate is from about 10 nanometers to about 1000 micrometers.
22. A method of embodiment 20, wherein the particle size of said particulate is from about 1 micrometer to about 100 micrometers.
23. A method of embodiment 20, wherein the second conductive material comprises from about 0.01 percent to about 10 percent, by weight, of the total weight of said particulate.
24. A method of embodiment 20, wherein said microoriganism is a bacteria.
25. A method of embodiment 20, wherein said bacteria is selected from MRAA and VRE.

## Claims

1. A composition comprising galvanic particulates for use in a method of treating excessive sweating or more specifically hyperhidrosis.

2. The composition of claim 1, wherein the galvanic particulates comprise a first conductive material and a second conductive material, wherein both said first conductive material and said second conductive material are exposed on the surface of said particulate, wherein the particle size of said particulate is from 10 nanometers to 100 micrometers, wherein the second conductive material comprises from 0.01 percent to 10 percent, by weight, of the total weight of said particulate, and wherein the difference of the standard potentials of the first conductive material and the second conductive material is at least 0.2 V.

3. The composition of claim 2, wherein said particulate comprises said first conductive material and wherein the surface of said particulate is partially coated with said second conductive material.

4. The composition of claim 2, wherein said particulate comprises at least 90 percent, or optionally at least 95 percent, by weight, of said first conductive material and said second conductive material.

5. The composition of claim 2, wherein;
a) said first conductive material is zinc;
b) said second conductive material is copper or silver; or
c) said first conductive material is zinc and said second conductive material is copper or silver.

6. The composition of any preceding claim, wherein said particulate is partially coated with a third conductive material.

7. The composition of claim 6, wherein said particulate comprises at least 95 percent, by weight, of said first conductive material, said second conductive material, and said third conductive material.

8. The composition of claim 7, wherein said second conductive material is copper and said third conductive material is silver.

9. The composition of any one of claims 3 - 8, wherein the composition further comprises a pharmaceutically-acceptable carrier.

10. The composition of any one of claims 3 - 9, wherein said first conductive material includes alloys of zinc, iron, aluminum, magnesium, copper and manganese or said second conductive material includes alloys of silver, copper, stainless steel and gold.

11. A galvanic particulate comprising a first conductive material and a second conductive material, wherein both said first conductive material and said second conductive material are exposed on the surface of said particulate, wherein the particle size of said particulate is from 10 nanometers to 100 micrometers, wherein the second conductive material comprises from 0.01 percent to 10 percent, by weight, of the total weight of said particulate, and wherein the difference of the standard potentials of the first conductive material and the second conductive material is at least 0.2 V.

12. Galvanic particulates according to claim 11, wherein the particulates are in flattened and/or elongated shapes, and wherein the longest dimension is at least twice the shortest dimension of such particulates.
